# EUROPÄISCHE PATENTSCHRIFT

(11) **EP 2 168 486 B1**
(45) Veröffentlichungstag und Bekanntmachung des Hinweises auf die Patenterteilung: **05.10.2011**
(21) Anmeldenummer: 09154891.7
(22) Anmeldetag: 11.03.2009
(51) Int. Cl.: A61B 6/00, A61B 6/03, A61B 6/04, A61B 19/00, A61B 17/34

(54) **Modulares System zur Brustdiagnose und -intervention**
Modular system for breast diagnosis and breast interventions
Système modulaire diagnostique et interventionelle sur le sein

(30) Priorität: 29.09.2008 DE 102008042430
(43) Veröffentlichungstag der Anmeldung: 31.03.2010
(73) Patentinhaber: MIR Medical Imaging Research Holding GmbH, 91096 Möhrendorf (DE); Friedrich-Alexander-Universität Erlangen-Nürnberg, 91054 Erlangen (DE)
(72) Erfinder: Kalender, Willi, 91096, Möhrendorf (DE); Schilling, Harry, 85072, Eichstätt (DE)
(74) Vertreter: Lohr, Georg

(56) Entgegenhaltungen:
- EP-A- 1 700 568
- WO-A-93/17620
- WO-A-2006/119426
- US-A- 5 569 266
- US-A- 5 803 912
- US-A1- 2007 064 867
- US-A1- 2008 089 471

## Beschreibung

### Technisches Gebiet

Die Erfindung betrifft ein Röntgengerät zur Abbildung der weiblichen Brust (Mammografie). Mit diesem Gerät können auch Eingriffe in die Brust vorgenommen werden.

### Stand der Technik

Zur Untersuchung der weiblichen Brust sind verschiedene Röntgengeräte bekannt. Unter einer Liege, auf der eine zu untersuchende Patientin liegt, befindet sich eine Röntgenvorrichtung mit einer rotierenden Gantry, welche eine Röntgenröhre und einem Detektor aufweist. Ein solches Gerät ist beispielsweise in der US 4,015,836 offenbart. Nachteilig an diesem Stand der Technik ist der große Platzbedarf so wie die mangelnde Zugänglichkeit der zu untersuchenden Brust. Weiterhin wird die Patientin in eine relativ unbequeme Lage mit tief liegendem Kopf gebracht, damit von der Röntgenvorrichtung ein möglichst großer Anteil der Brust erfasst werden kann. Eine Verbesserung stellt hier die Anordnung aus der US 2006/0094950 dar. Die Patientin hat hier eine bequemere Lage. Weiterhin ist auch die zu untersuchende Brust nur mit speziellen Instrumenten zugänglich. Nachteilig an dieser Anordnung ist der große Platzbedarf, der sich durch die große Bauform der Gantry ergibt. In der US 2007/0064867 ist ein Röntgengerät, nach dem Oberbegriff von Anspruch 1, basierend auf einem Spiral-CT Scanner offenbart. Hier wird die Auflösung durch die wenig stabile mechanische Konstruktion begrenzt. Zudem ist auch hier die zu untersuchende Brust nicht von außen zugänglich.

### Darstellung der Erfindung

Der Erfindung liegt die Aufgabe zugrunde, ein Röntgengerät zu gestalten, welches die weibliche Brust diagnostisch exakt, schnell und kostengünstig abbildet und gleichzeitig Eingriffe in die Brust ermöglicht. Gleichzeitig soll der behandelnde Arzt einen guten und ergonomisch vorteilhaften Zugang zu der Brust haben.

Diese Aufgabe wird durch ein Röntgengerät nach Anspruch 1 gelöst. Vorteilhafte Ausgestaltungen der Erfindung sind in den Unteransprüchen angegeben.

Ein erfindungsgemäßes Röntgengerät zur Abbildung einer weiblichen Brust umfasst eine Patientenliege 20, an welcher eine Gantry 10 eines Spiral- Computertomographen aufgehängt ist. Die Patientenliege 20 weist einen Brustausschnitt 21 auf, durch welchen die Brust 31 einer Patientin 30 vorzugsweise nach unten in Richtung der Gantry 10 hängt. Die Gantry 10 hat einen Gantryhubantrieb 11, mit dem sie gegenüber dem Patiententisch 20 bewegt werden kann. Zur Röntgenaufnahme rotiert die Gantry 10 um die Brust 31 der Patientin 30. Gleichzeitig und/oder in Zeitintervallen gestaffelt erfolgt eine Verschiebung der Gantry 10 in Längsrichtung der Brust, das heißt vorzugsweise in vertikaler Richtung. Die Verschiebung kann wahlweise kontinuierlich mit konstanter Geschwindigkeit oder proportional zur Rotation der Gantry erfolgen. Alternativ kann die Verschiebung auch in Schritten erfolgen, so dass beispielsweise nach jeder Umdrehung der Gantry ein Versatz um die Breite des Detektors 14 erfolgt.

Zur Intervention, insbesondere für eine Biopsie ist ein Instrument 123 vorgesehen, welches von unten in die freie Öffnung der Gantry eingreift. Damit können ohne die Gantry zu beeinflussen, Proben aus der Brust entnommen oder auch Kontrastmittel injiziert werden. Dieses Instrument befindet sich bevorzugt auf einem Instrumententräger 124, der wahlweise über einen Gelenkarm 126 mit der Patientenliege 20 oder der Gantryaufhängung 125 verbunden ist. Alternativ hierzu kann der Instrumententräger 124 auf einer mobilen Plattform 130 angeordnet sein. Besonders vorteilhaft ist es, wenn zur exakten Positionierung des Instrumententrägers dieser über einen Hexapod 121 mit der mobilen Plattform 130 verbunden ist. Zur exakten Positionierung des Instrumententrägers 124 sind Messeinrichtungen vorhanden. Im Falle eines Gelenkarms hat dieser in den Drehgelenken 123 bevorzugt Winkel- oder Positionssensoren. Weiterhin kann ein Messsystem vorgesehen sein, welches wahlweise die Position des Instrumententrägers oder auch der mobilen Plattform zur Gantry, dem Patiententisch oder einem anderen Fest damit verbundenen Teil ermittelt und entsprechende Korrekturinformationen an den Gelenkarm beziehungsweise den Hexapod übersendet.

Mit einem erfindungsgemäßen Röntgengerät können zunächst ohne den Instrumententräger bzw. das Interventionsinstrument Röntgenaufnahmen beziehungsweise Untersuchungen der Brust angestellt werden. Aufgrund der Konstruktion, bei der die Gantry an der Patientenliege aufgehängt ist, ergibt sich eine kompakte Platz sparende Bauweise, bei der auch der untersuchende beziehungsweise behandelnde Arzt oder anderes Personal Zugang zu der zu untersuchenden Brust. So kann beispielsweise vor einer Aufnahme oder zwischen zwei Aufnahmen ein Kontrastmittel in die Brust injiziert werden. Soll nun eine Intervention, wie eine Biopsie durchgeführt werden, so wird der Instrumententräger mit dem entsprechend Instrument unterhalb der Gantry eingebracht. Dieses besonders einfach, wenn hierzu eine Mobile Plattform vorhanden ist. Diese kann dann beispielsweise auf eigenen Rädern von einer seitlichen Position aus unter die Gantry gerollt werden. In den meisten Fällen wird eine exakte Justierung nicht notwendig sein, so dass einfache Markierungen auf dem Boden ausreichen. Eine exakte Justierung des Instrumententrägers mit dem Instrument erfolgt dann durch eine Positioniereinheit, die an der mobilen Plattform angebracht ist aufgrund von Positionsdaten, die mittels wenigstens eines Messsystems ermittelt wurden. Nach der Intervention, beispielsweise einer Biopsie kann die Mobile Plattform wieder aus dem Bereich der Gantry gefahren werden, so dass der untersuchende Arzt wieder Zugang zur Brust hat. Durch die Erfindungsgemäße Vorrichtung ist eine Umbettung der Patientin zwischen Röntgenaufnahme und Biopsie nicht notwendig. Weiterhin ist es nicht mehr notwendig, die Einstichpunkte auf der Haut entsprechend dem Röntgenbild zu markieren und dann durch diese die Biopsie vorzunehmen. Vielmehr kann die Biopsie nun exakt gesteuert aufgrund der Röntgendaten vorgenommen werden.

Entsprechend einer weiteren Ausgestaltung der Erfindung kann während einer Intervention die exakte Positionierung des Instruments überwacht werden. Hierzu werden während der Intervention eine oder mehrere Röntgenaufnahmen zur Kontrolle des Instruments beziehungsweise der Position des Instruments gemacht. Vorteilhafterweise dreht sich die Gantry kontinuierlich während der Intervention und macht um 90 Grad versetzte Aufnahmen. Dadurch ist das Interventionsinstrument in zwei Ebenen kontrollierbar. So kann beispielsweise festgestellt werden, ob sich eine Biopsienadel verbiegt.

Ein Verfahren, welches mit dem hier beschriebenen Röntgengerät durchgeführt werden kann, umfasst die folgenden Schritte:
- Erstellen einer dreidimensionalen Röntgenaufnahme,
- Erfassen der relativen Position des Interventionsinstrumentes (122) in Bezug auf die Gantry (10),
- Berechnen der Position des Interventionsinstrumentes (122) in Bezug auf die dreidimensionale Röntgenaufnahme.

Bei diesem Verfahren können die ersten beiden Schritte in ihrer Reihenfolge vertauscht werden. Wesentlich hierbei ist die Berechnung der Position des Interventionsinstrumentes (122) mittels seiner relativen Position in Bezug auf die Gantry oder eines mit der Gantry verbundenen Teils.

### Beschreibung der Zeichnungen

Die Erfindung wird nachstehend ohne Beschränkung des allgemeinen Erfindungsgedankens anhand von Ausführungsbeispielen unter Bezugnahme auf die Zeichnungen exemplarisch beschrieben.
Figur 1 zeigt eine Erfindungsgemäße Vorrichtung
Figur 2 zeigt eine Positioniereinheit mit einem Hexapod
Figur 3 zeigt eine Erfindungsgemäße Vorrichtung in seitlicher Ansicht
Figur 4 zeigt eine Erfindungsgemäße Vorrichtung mit einem Gelenkarm

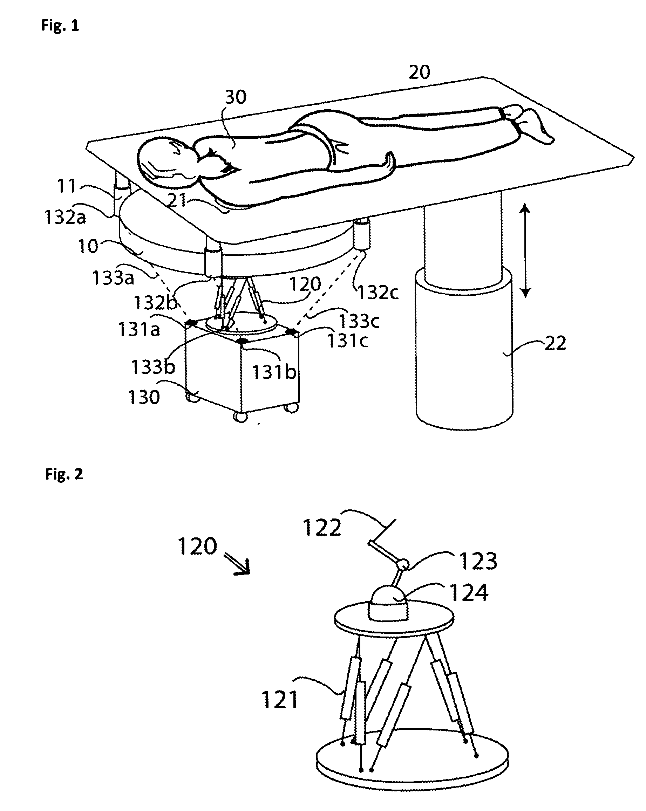

In Figur 1 ist ein erfindungsgemäßes Röntgengerät dargestellt. Auf der Patientenliege 20 liegt eine Patientin 30. Die zu untersuchende Brust hängt über einen Brustausschnitt 21 durch die Patientenliege 20 in den Aufnahmebereich einer Gantry 10. Die Gantry 10 ist eine Spiral- Computertomographen Gantry mit einer Röntgenröhre und einem Detektor, welche sich um die zu untersuchende Brust drehen. Während der Drehung wird die Brust abgebildet. Gleichzeitig mit der Drehung wird über den Gantryhubantrieb 11 eine Verschiebung in vertikaler Richtung durchgeführt, so dass die Brust spiralförmig abgetastet wird. Die Patientenliege 20 ist über einen Patientenliegenhubantrieb 22 in der Höhe verstellbar. Optional kann bei einem fest installierten Patiententisch dieser auch noch um die Achse des Patientenliegenhubantriebs 22 drehbar sein.

Unter der Gantry 10 befindet sich eine Mobile Plattform 130. Diese Mobile Plattform kann während der Untersuchung unter die Gantry gefahren oder auch wieder entfernt werden. Sie weist bevorzugt Räder auf, die vorteilhafterweise blockierbar sind. Wahlweise kann sie auf dem Fußboden oder aber auch auf Schienen rollen oder gleiten. Besonders günstig Gleiter oder Luftgleiter basierend auf Luftkissenlagern. Die Mobile Plattform kann einen eigenen Antrieb zur exakten Positionierung unterhalb der Gantry aufweisen oder auch manuell dorthin verschoben werden. Führungsschienen oder auch in den Boden eingebettete Positionierhilfen wie Induktionsschleifen können eine exakte Positionierung vereinfachen. Der in dieser Figur durch die Gantry verdeckte Instrumententräger wird mittels einer Positioniereinheit 120 in seiner Arbeitsposition an der Brust gehalten. Die Positioniereinheit kann beispielsweise ein Hexapod oder auch ein Gelenkarm sein. Zur exakten Positionierung sind Positionsmesssysteme 131a, 131b und 131c vorgesehen, die mit Messsignalen 133a, 133b, 133c, hier beispielsweise Licht, den Abstand zu Messmarken 132a, 132b, 132c an der Gantryaufhängung ermitteln. Wahlweise kann nun aufgrund der Messung der Positionsmesssysteme eine automatische oder auch eine manuelle Korrektur der Position der mobilen Plattform erfolgen. Alternativ und/oder zusätzlich können die Positionsdaten des Messsystems zur Berechnung der exakten Position des Instrumententrägers herangezogen werden. Das Messsystem kann grundsätzlich nach allen bekannten Prinzipien zur Positions- beziehungsweise Distanzmessung arbeiten. Bevorzugt arbeitet es mit optischen Sensoren wie Lasersensoren oder auch mit Ultraschallsensoren oder Funksensoren.

In Figur 2 ist eine Positioniereinheit 120 dargestellt, die den Instrumententräger 124 in die richtige Arbeitsposition in Bezug auf die Brust positioniert. Die exakte Positionierung erfolgt hier mittels eines Hexapod 121. Vorteilhafterweise ist zwischen den Instrumententräger und dem Interventions-Instrument 122 noch ein Gelenkarm mit wenigstens einem Drehgelenk 123 vorgesehen. Grundsätzlich können auch mehrere Gelenke vorgesehen sein. Aufgrund des Hexapod ist dies in der dargestellten Ausführungsform meist nicht notwendig.

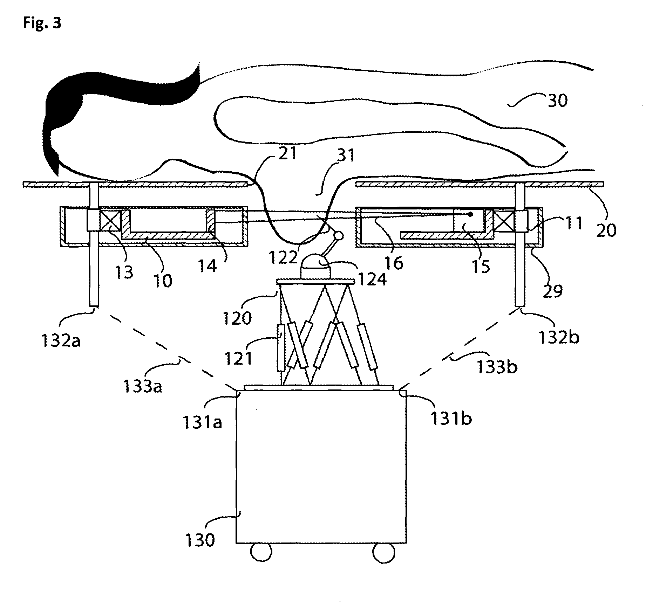

In Figur 3 ist eine erfindungsgemäße Vorrichtung in seitlicher Ansicht dargestellt. Zur besseren Veranschaulichung ist noch die Gantry im Schnitt dargestellt. Innerhalb des Gantrygehäuses 29 befindet sich eine Röntgenröhre 15, welche einen Strahlenfächer 16 zur Durchstrahlung der Brust 31 erzeugt. Die Strahlung wird von einem Detektor 14 empfangen und zu einer Auswerteeinheit (hier nicht dargestellt) geführt. Die Gantry ist einerseits mit einem Gantrydrehlager 13 um die Brust drehbar und über den Gantryhubantrieb 11 in ihrer Höhe beziehungsweise im inneren Abstand zur Patientin verschiebbar. Damit ist eine spiralförmige Abtastung der zu untersuchenden Brust 31 möglich.

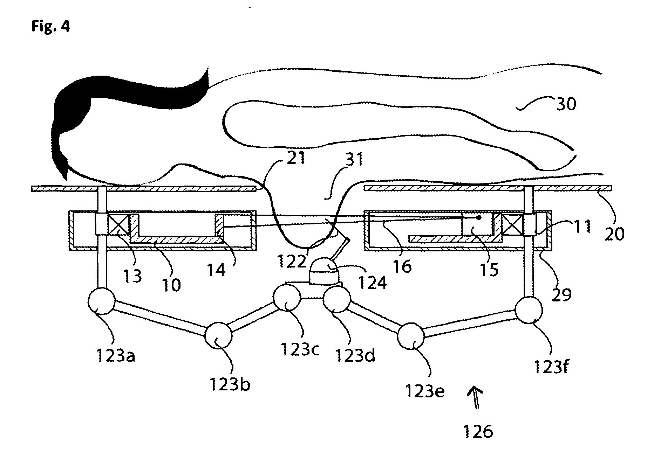

In Figur 4 ist eine erfindungsgemäße Vorrichtung mit einem Gelenkarm zur Positionierung des Instrumententrägers 124 und des Intervention- Instruments 122 offenbart. Dieser Gelenkarm ist hier an den Trägern der Gantryaufhängung 125 fixiert. Vorteilhafterweise lässt er sich während der Untersuchung beziehungsweise Behandlung entfernen oder auch wieder an der Gantryaufhängung befestigen. Anstelle der Gantryaufhängung kann der Gelenkarm auch an der Patientenliege 20 selbst oder einem anderen damit verbundenen Bauteil befestigt sein. Vorzugsweise ist eine solche Befestigung auf einfache Art und Weise, beispielsweise mit einem Schnellverschluss lösbar, so dass die gesamte Baugruppe mit dem Gelenkarm 126 schnell montiert und auch wieder abgenommen werden kann. Er kann auch auf einer mobilen Plattform 130, wie in Figur 1 dargestellt, anstelle des dort gezeichneten Hexapod eingesetzt werden. Der hier dargestellte Gelenkarm weist mehrere Drehgelenke 123a, 123b, 123c, 123d, 123e und 123f auf. In den Drehgelenken sind vorteilhafterweise Drehantriebe und/oder Winkelsensoren und/oder Positionssensoren integriert. Grundsätzlich könnte auch ein Messsystem an den Instrumententräger vorhanden sein, so das die exakte Position des Instrumententräger in Bezug auf die Gantry 10 und/oder die Patientenliege 20 ermittelt werden kann.

### Bezugszeichenliste

- 10: Gantry
- 11: Gantryhubantrieb
- 13: Gantrydrehlager
- 14: Detektor
- 15: Röntgenröhre
- 16: Strahlenfächer
- 20: Patientenliege
- 21: Brustausschnitt
- 22: Patientenliegenhubantrieb
- 29: Gantrygehäuse
- 30: Patient
- 31: Brust
- 120: Positioniereinheit
- 121: Hexapod
- 122: Interventions- Instrument
- 123: Drehgelenk
- 124: Instrumententräger
- 125: Gantryaufhängung
- 126: Gelenkarm
- 130: Mobile Plattform
- 131: Messsystem
- 132: Messmarke
- 133: Messsignal (optisch)

## Patentansprüche

1. Röntgengerät zur Abbildung und Intervention einer Brust einer Patientin (30), mit
- einer Röntgeneinrichtung mit einer näherungsweise um eine vertikale Drehachse drehbare Gantry (10) umfassend eine Röntgenröhre (15) und einen Röntgendetektor (14), wobei die Gantry (10) mittels eines Gantryhubantriebs (11) in vertikaler Richtung bewegt werden kann und die vertikale Bewegung in Abhängigkeit von der Rotationsbewegung erfolgt, und
- einer horizontal angeordneten Patientenliege (20) mit einem Brustausschnitt (21) zur Aufnahme einer Brust der Patientin (30),
**dadurch gekennzeichnet, dass**
die Gantry (10) an der Unterseite der Patientenliege (20) aufgehängt ist und ein Instrumententräger (124) zur Aufnahme wenigstens eines Interventions-Instruments (122) vorgesehen ist, der von unten durch die Gantry in der Umgebung der Brust positioniert wird, so dass mit dem Interventions-Instrument eine Intervention ausgeführt werden kann.

2. Röntgengerät nach Anspruch 1,
**dadurch gekennzeichnet, dass**
eine mobile Plattform (130) vorgesehen ist, welche über eine Positioniereinheit (120) den Instrumententräger (124) trägt, wobei die Mobile Plattform (130) von der Seite unter die Gantry (10) eingebracht werden kann.

3. Röntgengerät nach Anspruch 1,
**dadurch gekennzeichnet, dass**
ein Gelenkarm (126) vorgesehen ist, welcher den Instrumententräger (124) trägt, und an der Gantryaufhängung (125) oder anderen fest mit dieser verbundenen Teilen fixiert ist.

4. Röntgengerät nach Anspruch 3,
**dadurch gekennzeichnet, dass**
die Fixierung des Gelenkarms mit einer Schnellverschlusseinrichtung lösbar ist.

5. Röntgengerät nach Anspruch 2,
**dadurch gekennzeichnet, dass**
die Mobile Plattform (130) wenigstens ein Messsystem (131) zur exakten Bestimmung der Position der mobilen Plattform gegenüber der Gantry (10) oder eines fest mit dieser verbundenen Teiles aufweist, wobei die durch das Messsystem bestimmten Positionsinformationen zur exakten Positionierung des Instrumententräger (124) verwendet werden.

6. Röntgengerät nach Anspruch 2,
**dadurch gekennzeichnet, dass**
die Positioniereinheit (120) ein Hexapod (121) ist.

7. Röntgengerät nach Anspruch 2,
**dadurch gekennzeichnet, dass**
die Mobile Plattform (130) Räder, Gleiter oder Luftgleiter aufweist.

8. Röntgengerät nach Anspruch 1, 2 oder 3,
**dadurch gekennzeichnet, dass**
wenigstens ein Messsystem (131) zur Bestimmung der Position des Instrumententrägers (124) oder eines damit verbundenen Teils gegenüber der Gantry (10) oder eines fest mit dieser verbundenen Teiles Positionsinformationen zur exakten Positionierung des Instrumententrägers (124) und/oder des Instruments (122) in Bezug auf die Brust (31) liefert.

9. Röntgengerät nach einem der vorhergehenden Ansprüche,
**dadurch gekennzeichnet, dass**
während einer Intervention Röntgenaufnahmen zur Kontrolle der Position des Interventions- Instruments (122) durchgeführt werden.

## Claims

1. X-ray machine for imaging and performing surgical intervention on the breast of a female patient (30), comprising:
- an X-ray facility with a gantry (10) that is rotatable approximately about a vertical rotation axis and comprises an X-ray tube (15) and an X-ray detector(14), with the gantry (10) being adapted to be moved in a vertical direction by means of a gantry lift drive (11), and a vertical movement of the gantry being effected in dependence upon the rotational movement; and
- a horizontally disposed patient's table (20) having a cutout portion (21) for a breast, for accommodating a patient's breast (30);
**characterized in that**
the gantry (10) is suspended from a lower side of the patient's table (20), and an instrument holder (124) for accommodating at least one operating instrument (12) is provided which is positioned in the vicinity of the breast through the gantry from below, so that a surgical intervention can be effected with the operating instrument.

2. X-ray machine according to claim 1,
**characterized in that**
a mobile platform (130) is provided for supporting the instrument holder (124) via a positioning unit (120), with the mobile platform (130) being adapted to be inserted underneath the gantry from a side.

3. X-ray machine according to claim 1,
**characterized in that**
an articulated arm (126) is provided which supports the instrument holder (124) and is fastened to a gantry suspension (125) or other components firmly connected to the gantry suspension.

4. X-ray machine according to claim 3,
**characterized in that**
fastening of the articulated arm is adapted to be unfastened with a rapid closure means.

5. X-ray machine according to claim 2,
**characterized in that**
the mobile platform (130) comprises at least one measuring system (131) for exactly determining a position of the mobile platform relative to the gantry (10) or another component firmly connected to the gantry, with position information determined with the measuring system being used for exact positioning of the instrument holder (124).

6. X-ray machine according to claim 2,
**characterized in that**
the positioning unit (120) is a hexapod (121).

7. X-ray machine according to claim 2,
**characterized in that**
the mobile platform (130) is provided with wheels, slides or air bearings.

8. X-ray machine according to claim 1, 2 or 3
**characterized in that**
at least one measuring system (131) for determining a position of the instrument holder (124) or of a component connected thereto relative to the gantry (10) or a component firmly connected thereto is adapted to supply position information for exactly positioning at least one of the instrument holder (124) and the instrument (122) relative to the breast.

9. X-ray machine according to anyone of the preceding claims,
**characterized in that**
X-ray exposures are taken during a surgical intervention to check a position of a surgical intervention instrument (12).

## Revendications

1. Appareil radiographique pour l'imagerie et l'intervention sur un sein d'une patiente (30), avec
- un dispositif radiographique avec un portique (10) pouvant tourner approximativement autour d'un axe de rotation vertical et comprenant un tube à rayons X (15) et un détecteur de rayons X (14), le portique (10) pouvant être déplacé dans le sens vertical au moyen d'un entraînement de levage du portique (11) et le mouvement vertical s'effectuant en fonction du mouvement de rotation, et
- une couchette (20) horizontale avec une découpe pour un sein (21) destinée à recevoir un sein de la patiente (30),
**caractérisé en ce que** le portique (10) est suspendu à la face inférieure de la couchette (20) et un support d'instrument (124) est prévu pour recevoir au moins un instrument d'intervention (122) positionné au voisinage du sein en passant par dessous à travers le portique, de sorte qu'une intervention peut être réalisée avec l'instrument d'intervention.

2. Appareil radiographique selon la revendication 1, **caractérisé en ce qu'**il est prévu une plate-forme mobile (130) qui porte le support d'instrument (124) par l'intermédiaire d'une unité de positionnement (120), la plate-forme mobile (130) pouvant être introduite par le côté sous le portique (10).

3. Appareil radiographique selon la revendication 1, **caractérisé en ce qu'**il est prévu un bras articulé (126) qui porte le support d'instrument (124) et qui est fixé à la suspension du portique (125) ou à d'autres pièces reliées de manière fixe à celle-ci.

4. Appareil radiographique selon la revendication 3, **caractérisé en ce que** la fixation du bras articulé peut être défaite à l'aide d'un dispositif à fermeture rapide.

5. Appareil radiographique selon la revendication 2, **caractérisé en ce que** la plate-forme mobile (130) comporte au moins un système de mesure (131) pour la détermination exacte de sa position par rapport au portique (10) ou à une pièce reliée de manière fixe à celui-ci, les informations de position déterminées par le système de mesure étant utilisées pour le positionnement exact du support d'instrument (124).

6. Appareil radiographique selon la revendication 2, **caractérisé en ce que** l'unité de positionnement (120) est un hexapode (121).

7. Appareil radiographique selon la revendication 2, **caractérisé en ce que** la plate-forme mobile (130) présente des roues, des patins ou des coussins d'air.

8. Appareil radiographique selon la revendication 1, 2 ou 3, **caractérisé en ce qu'**au moins un système de mesure (131) pour déterminer la position du support d'instrument (124) ou d'une pièce reliée à celui-ci par rapport au portique (10) ou à une pièce reliée de manière fixe à celui-ci donne des informations de position pour le positionnement exact du support d'instrument (124) et/ou de l'instrument (122) par rapport au sein (31).

9. Appareil radiographique selon l'une des revendications précédentes, **caractérisé en ce que** pendant une intervention, des clichés radiographiques sont réalisés afin de contrôler la position de l'instrument d'intervention (122).
